(19) Europäisches Patentamt
European Patent Office
Office européen des brevets

(11) **EP 3 508 121 A1**

(12) **EUROPEAN PATENT APPLICATION**

(43) Date of publication:
**10.07.2019 Bulletin 2019/28**

(51) Int Cl.:
**A61B 5/11** *(2006.01)*          *A61B 5/0402 (2006.01)*
**A61B 5/00** *(2006.01)*          *G01C 25/00 (2006.01)*

(21) Application number: **18150136.2**

(22) Date of filing: **03.01.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Koninklijke Philips N.V.**
**5656 AE Eindhoven (NL)**

(72) Inventors:
• **RISPENS, Sietse**
**5656 AE Eindhoven (NL)**
• **BONOMI, Alberto Giovanni**
**5656 AE Eindhoven (NL)**
• **DERKX, Rene Martinus Maria**
**5656 AE Eindhoven (NL)**
• **AARTS, Vincent Alexander Rudolf**
**5656 AE Eindhoven (NL)**
• **HUIJBREGTS, Laurentia Johanna**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DEVICE FOR DETERMINING A TRANSFORMATION BETWEEN A COORDINATE SYSTEM OF AN ACCELEROMETER AND A COORDINATE SYSTEM OF A SUBJECT**

(57)    The invention relates to a device for determining a transformation between an accelerometer coordinate system of an accelerometer 3 attached to a subject and a subject coordinate system. Defining that a vertical and upward pointing direction in the subject coordinate system is equal to an acceleration-measurement-based vertical and upward pointing direction in the accelerometer coordinate system leads to a first direction in the accelerometer coordinate system. A second direction in the accelerometer coordinate system is provided, which is equal to a direction in the subject coordinate system of the subject having a known spatial relation to the vertical and upward pointing direction in the subject coordinate system, wherein the transformation is determined based on the first and second directions and the known spatial relation. This allows for an improved determination of the transformation, which can be used for, for instance, improved activity or posture detection.

FIG. 3

EP 3 508 121 A1

**Description**

FIELD OF THE INVENTION

The invention relates to a device, a method and a computer program for determining a transformation between a coordinate system of an accelerometer attached to a subject and a coordinate system of the subject. The invention relates further to a system, a method and a computer program for determining a condition of a subject, i.e., for instance, a subject's posture and/or activity like sleeping, walking, cycling et cetera.

BACKGROUND OF THE INVENTION

Accelerometers are sensors which are adapted to measure inertial accelerations as well as gravitational accelerations. An accelerometer attached to a subject such as a person can therefore be used to determine, for instance, the subject's posture. In order to reliably determine the subject's posture based on the measured acceleration, it is generally required to know a transformation between a coordinate system of the accelerometer and a coordinate system of the subject and hence the spatial relation between these two coordinate systems, wherein a determination of such a transformation is regarded as being a calibration.

US 9,035,794 B2 discloses such a calibration, wherein the calibration described in this US document does not accurately consider the shape of the part of the subject, to which the accelerometer is attached, i.e., for instance, it does not consider the shape of the chest of the subject in the sagittal plane, if the accelerometer is attached to the chest. This can lead to an inaccurate calibration and hence to an inaccurate determination of a subject's condition like the subject's posture.

SUMMARY OF THE INVENTION

It is an object of the present invention to provide a device, a method and a computer program which allow for an improved determination of a transformation between a coordinate system of an accelerometer attached to a subject and a coordinate system of the subject. Moreover, it is an object of the present invention to provide a system, a method and a computer program for determining a condition of a subject like the subject's posture and/or the subject's activity.

In a first aspect of the present invention a device for determining a transformation between a coordinate system of an accelerometer attached to a subject and a coordinate system of the subject is presented, wherein the device comprises:

a transformation determination unit adapted to determine a transformation between the coordinate system of the accelerometer and the coordinate system of the subject, wherein the determination of the transformation includes:

a) determining a vertical and upward pointing direction in the coordinate system of the accelerometer based on an acceleration measured by the accelerometer, providing a vertical and upward pointing direction in the coordinate system of the subject and defining that the vertical and upward pointing direction in the coordinate system of the subject is equal to the vertical and upward pointing direction in the coordinate system of the accelerometer, in order to define a first direction in the coordinate system of the accelerometer,
b) providing a second direction in the coordinate system of the accelerometer being equal to a direction in the coordinate system of the subject having a known spatial relation to the vertical and upward pointing direction in the coordinate system of the subject,
c) determining the transformation based on the first and second directions and the known spatial relation.

Since it is defined that the vertical and upward pointing direction in the coordinate system of the subject is equal to the vertical and upward pointing direction in the coordinate system of the accelerometer, in order to define a first direction in the coordinate system of the accelerometer, and since the second direction in the coordinate system of the accelerometer is provided such that it is equal to a direction in the coordinate system of the subject having a known spatial relation to the vertical and upward pointing direction in the coordinate system of the subject, wherein the transformation is determined based on the first and second directions and the known spatial relation, the determined transformation is not adversely influenced by a shape of the part of the subject, to which the accelerometer is attached. The determination of the transformation is therefore improved.

In order to determine the vertical and upward pointing direction in the coordinate system of the accelerometer, preferentially the acceleration is measured, while the part of the subject, to which the accelerometer is attached, is not moved, at least not voluntarily moved. The measured acceleration is then mainly caused by the gravitational field and hence vertical and upward pointing and defines the desired direction. For instance, if the accelerometer is attached to the subject's chest, this direction is determined, if the chest of the subject is not voluntarily moved. However, the chest is of course slightly involuntarily moved due to, for instance, breathing or cardiac motion, wherein this involuntary movement is so small that the measured acceleration is still substantially a vertical and upward pointing acceleration which can be used for reliably determining the transformation. The voluntary movement, which should not be present when determining this direction, can be an active movement, i.e. the subject can actively move, or a passive movement, i.e. the subject can be moved by, for instance, a nurse who might move the subject or by a device like a bed, on which the subject might be lying, wherein the subject might be voluntarily passively moved by changing a reclination angle of the bed. Thus, this direction is preferentially determined if the subject does not voluntarily, actively or passively move the accelerometer. The device can comprise a user interface allowing a user to indicate when the part of the subject, to which the accelerometer is attached, does not move, i.e. at least not voluntarily move. The device can also comprise another means for detecting this non-movement. The transformation determination unit can be adapted to determine the transformation, if it is indicated that the part of the subject, to which the accelerometer is attached, does not move, i.e. at least not voluntarily move.

The transformation determination unit is preferentially adapted to provide the second direction such that it is equal to the medial-lateral direction in the coordinate system of the subject. This can simplify the determination of the transformation, if, for instance, an axis of the coordinate system of the subject is defined by the medial-lateral direction. In an embodiment a housing, in which the accelerometer is preferentially mounted, has an easily recognizable axis, wherein the position of this axis is known in the coordinate system of the accelerometer. For instance, this easily recognizable axis can coincide with an axis of the coordinate system of the accelerometer, which might be named $y$ axis. A user like a nurse can be advised to attach the housing to the subject such that this easily recognizable axis of the housing is aligned with the medial-lateral direction of the subject. The transformation determination unit can then be adapted to provide the second direction, i.e. in this example the medial-lateral direction, in the coordinate system of the accelerometer based on the known position of the easily recognizable axis on the housing in the coordinate system of the accelerometer. In another embodiment the second direction can be another direction, i.e. not the medial-lateral direction, which is linearly independent of the first direction.

The subject is preferentially a person. However, the subject can also be an animal or a technical object like a robot.

The transformation determination unit is preferentially adapted to determine the transformation based on the first and second directions such that it includes modifying the first and second directions such that they correspond to the known spatial relation and determining the transformation based on the modified first and second directions and the known spatial relation. Moreover, in a preferred embodiment the known spatial relation is an orthogonal spatial relation, wherein the transformation determination unit is adapted to modify the first and second directions such that they are orthogonal to each other. For instance, the second direction can be defined such that it is equal to the medial-lateral direction in the coordinate system of the subject, wherein the subject can be positioned such that the medial-lateral direction is perpendicular to the vertical direction. In particular, the subject or a person, who positions the subject, like a nurse can be instructed to position the subject such that the medial-lateral direction is perpendicular to the vertical direction. This can be ensured, for instance, by positioning the subject such that the subject is lying on his/her back. By modifying the first and second directions such that they correspond to the known spatial relation, particularly are orthogonal to each other, and by then determining the transformation based on the modified first and second direction and the known spatial relation, the accuracy of the determination of the transformation and hence of a spatial relation between the coordinate system of the accelerometer and the coordinate system of the subject can be further improved.

It is preferred that the transformation determination unit is further adapted to determine, before modifying the first and second directions such that they correspond to the known spatial relation, whether a deviation from the known spatial relation is smaller than a predefined threshold and to stop the determination of the transformation, if the deviation is not smaller than the predefined threshold. If the deviation from the known spatial relation, i.e., for instance, from orthogonality, of the first and second directions with respect to each other is larger than the predefined threshold, it can be assumed that the current posture of the subject is such that the transformation and hence a spatial relationship between the coordinate systems of the accelerometer and the subject might not be accurately enough determined. In this case it is preferred that the spatial transformation and hence the spatial relation is not determined. The device can also comprise an output unit adapted to output a signal indicating that the transformation will not be determined in the current posture of the subject. If the subject or a person positioning the subject like a nurse is informed accordingly, the subject's posture might be modified such that, for instance, the medial-lateral direction is at least roughly orthogonal to the vertical direction, whereupon the determination of the transformation, i.e. the calibration, can be tried again. Alternatively, the nurse can be instructed to replace the device on a new location on the body of the subject such that the deviation from the known spatial relation is made smaller.

The device can comprise an electrocardiography signal providing unit adapted to generate an electrocardiography signal of the subject having a polarity sign, wherein the electrocardiography signal can be generated by an electrocardiography signal generating unit having a fixed spatial relation to the accelerometer, wherein the transformation determination unit can be adapted to provide the sign of the second direction based on the provided polarity sign. Thus, in an embodiment the polarity sign of the electrocardiography signal can be used for ensuring that the second direction really is, for instance, a medial-lateral direction of the subject in the coordinate system of the accelerometer, even if, for example, the accelerometer has been attached to the subject upside down, wherein this upside down arrangement of the accelerometer can be detected based on the polarity sign of the electrocardiography signal. In particular, a user like a nurse or the subject him/herself can be asked to attach the accelerometer to the subject such that an axis of the coordinate system of the accelerometer points in the medial-lateral direction, i.e. such that this axis defines the second direction in the coordinate system of the accelerometer. If the accelerometer has been attached to the subject in this way and not upside down, the provided electrocardiography signal has a first polarity, wherein, if the accelerometer has been attached to the subject upside down, this axis of the coordinate system of the accelerometer points in an opposite direction and the provided electrocardiography signal has an opposing polarity. In this case the second direction can be defined as being opposite to the direction of this axis. Thus, by using the electrocardiography signal for determining the sign of the second direction, it can be ensured that the second direction is correctly defined, even if the accelerometer is placed upside down on the subject. This can further improve the determination of the transformation and hence the calibration. Alternatively or in addition, the transformation determination unit can be adapted to determine the sign of the second direction based on an acceleration value received from the accelerometer. In particular, the acceleration value can be used for determining whether the accelerometer has been attached to the subject upside down, wherein in this case the sign of the second direction in the coordinate system of the accelerometer is preferentially changed. Also this allows for an accurate determination of the transformation, even if the accelerometer has been attached to the subject upside down.

The device can further comprise a posture providing unit adapted to provide the posture of the subject, wherein the transformation determination unit can be adapted to provide the vertical and upward pointing direction in the coordinate system of the subject based on the provided posture. The posture providing unit can be adapted to provide a user interface for allowing a user to input the posture of the subject into the device and/or to receive a user input indicating the posture of the subject from an input device at which the user has input the posture, wherein the posture providing unit is adapted to provide the input posture. Moreover, the posture providing unit can be adapted to provide a predefined stored posture as the posture of the subject. In particular, the accelerometer can be adapted to be attached to a chest of the subject, wherein the posture providing unit can be adapted to provide the posture of the subject by providing an angle between the subject's back and a horizontal plane, while the subject lies on the subject's back, wherein the transformation determination unit can be adapted to determine the vertical and upward pointing direction in the coordinate system of the subject in the sagittal plane based on the provided angle between the subject's back and the horizontal plane.

The coordinate system of the subject is preferentially defined by a medial-lateral direction, a caudal-cranial direction and an anterior-posterior direction such that the transformation determination unit is adapted to determine the transformation between the coordinate system of the accelerometer and the medial-lateral direction, the caudal-cranial direction and the anterior-posterior direction of the subject.

In a further aspect of the present invention a system for determining a condition of a subject is presented, wherein the system comprises:

an accelerometer adapted to be attached to the subject and to measure a first acceleration, while the subject does not move, and a second acceleration,
a device as defined in claim 1 for determining a transformation between a coordinate system of the accelerometer and a coordinate system of the subject, when the accelerometer is attached to the subject, wherein the first acceleration is used for determining the vertical and upward pointing direction in the coordinate system of the accelerometer, and
a condition determination unit for determining a condition of the subject based on the measured second acceleration and the determined transformation.

The condition determination unit is preferentially adapted to determine at least one of a moving state, a posture and an activity of the subject, i.e. the moving state and/or the posture and/or the activity, as the condition. The determination of the condition can also include whether the subject has fallen and/or it can include a chair rise detection.

In another aspect of the present invention a method for determining a transformation between a coordinate system of an accelerometer attached to a subject and a coordinate system of the subject is presented, wherein the method comprises:

determining a transformation between the coordinate system of the accelerometer and the coordinate system of the subject by a transformation determination unit, wherein the determination of the transformation includes:

a) determining a vertical and upward pointing direction in the coordinate system of the accelerometer based on an acceleration measured by the accelerometer, providing a vertical and upward pointing direction in the coordinate system of the subject and defining that the vertical and upward pointing direction in the coordinate system of the subject is equal to the vertical and upward pointing direction in the coordinate system of the accelerometer, in order to define a first direction in the coordinate system of the accelerometer,

b) providing a second direction in the coordinate system of the accelerometer being equal to a direction in the coordinate system of the subject having a known spatial relation to the vertical and upward pointing direction in the coordinate system of the subject,

c) determining the transformation based on the first and second directions and the known spatial relation.

In an aspect of the present invention a method for determining a condition of a subject is presented, wherein the method comprises:

measuring a first acceleration by an accelerometer attached to the subject, while the subject does not move, determining a transformation between a coordinate system of the accelerometer and a coordinate system of the subject based on the measured first acceleration as defined in claim 12, wherein the measured first acceleration is used for determining the vertical and upward pointing direction in the coordinate system of the accelerometer, measuring a second acceleration by the accelerometer attached to the subject, determining a condition of the subject based on the measured second acceleration and the determined transformation by a condition determination unit.

In a further aspect of the present invention a computer program for determining a transformation between a coordinate system of an accelerometer attached to a subject and a coordinate system of the subject is presented, wherein the computer program comprises program code means for causing a device as defined in claim 1 to carry out the steps of the method as defined in claim 12, when the computer program is run on a computer controlling the device.

In another aspect of the present invention a computer program for determining a condition of a subject is presented, wherein the computer program comprises program code means for causing a system as defined in claim 11 to carry out the steps of the method as defined in claim 13, when the computer program is run on a computer controlling the system.

It shall be understood that the device for determining a transformation as defined by claim 1, the system for determining a condition as defined by claim 11, the method for determining a transformation as defined by claim 12, the method for determining a condition as defined by claim 13, the computer program for determining a transformation as defined by claim 14, and the computer program for determining a condition as defined by claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 illustrates schematically and exemplarily how a system for determining a condition of a subject like its posture and/or its activity is placed on the left side of the chest of the subject,
Fig. 2 shows schematically and exemplarily the system for determining a condition of a subject and a coordinate system of an accelerometer located within the system,
Fig. 3 illustrates schematically and exemplarily several devices and units arranged within the system for determining a condition of a subject,
Fig. 4 shows schematically and exemplarily a coordinate system of the subject,
Fig. 5 shows schematically and exemplarily a bed, on which a person is lying, with a bed angle of 30°,
Fig. 6 illustrates schematically and exemplarily two directions within a sagittal plane of the person lying on the bed,
Fig. 7 shows a flowchart exemplarily illustrating an embodiment of a method for determining a transformation between the coordinate system of the accelerometer and the coordinate system of the subject,
Fig. 8 shows a flowchart exemplarily illustrating an embodiment of a method for determining a condition of a subject,
Fig. 9 shows schematically and exemplarily an electrocardiography signal of the system if it has been placed on the subject correctly, i.e. not upside down, wherein in this case the polarity, i.e. the QRS polarity, is positive, and
Fig. 10 shows schematically and exemplarily an electrocardiography signal if the system has been placed on the subject upside down, wherein in this case the polarity is negative.

DETAILED DESCRIPTION OF EMBODIMENTS

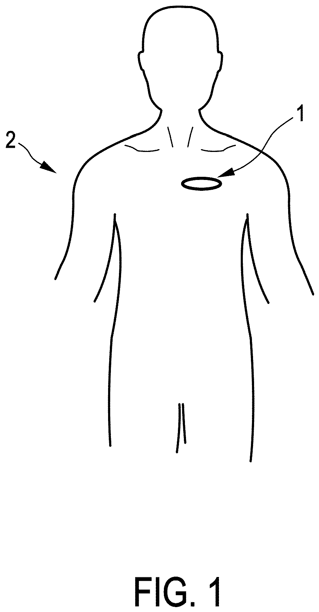

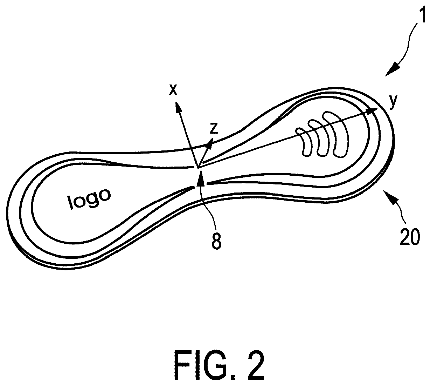

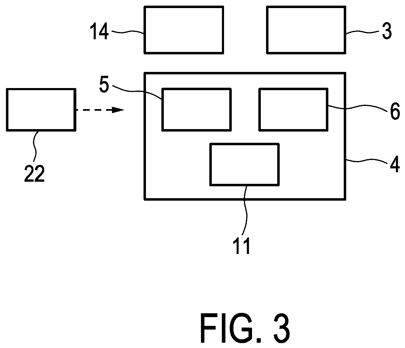

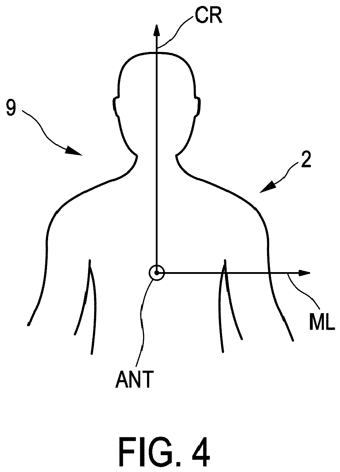

Fig. 1 shows a person 2 with a system 1 for determining a condition of the person 2. In this embodiment the system 1 is adapted to work properly, if it is attached horizontally on the left breast as schematically and exemplarily indicated in Fig. 1. The system 1 is schematically and exemplarily shown in more detail in Fig. 2. The system 1 comprises a housing 20 which can be attached to the person's left breast by using, for instance, adhesive. Within the housing 2 several electrical components are arranged, which are schematically and exemplarily shown in Fig. 3. For instance, the system 1 comprises an accelerometer 3 adapted to measure an acceleration, i.e. the inertial accelerations caused by motion of the subject and the gravitational accelerations from the earth's gravitational field, in its coordinate system 8 having three orthogonal axes x, y, z as schematically and exemplarily shown in Fig. 2. Within the housing 20 the accelerometer 3 is arranged such that the x axis of the coordinate system 8 is aligned with the width direction of the housing 20, the y axis is aligned with the length direction of the housing 20 and the z axis is aligned with a height direction of the housing 20, wherein the housing is elongated and has the length direction in the direction of the elongation. Also for the person 2 a coordinate system is defined, which in this embodiment is defined as illustrated in Fig. 4. The coordinate system 9 of the person 2 has a medial-lateral axis ML, a caudal-cranial axis CR and a posterior-anterior axis ANT.

The system 1 further comprises a device 4 for determining a spatial relation between the coordinate system 8 of the accelerometer 3 and the coordinate system 9 of the person 2, when the accelerometer 3 is attached to the person 2 as shown in Fig. 1. The device 4 comprises a posture providing unit 5 adapted to provide the posture of the person 2. In this embodiment the posture providing unit 5 is adapted to receive a user input indicating the posture of the person 2 from an input device 22 at which the user has input the posture. For instance, the input device 22 can be a mobile computer or a stationary computer with input means like a keyboard, a touch-sensitive surface, a computer mouse, et cetera. In another embodiment the posture providing unit can also be a storing unit in which a predefined stored posture is stored and which is provided by the posture providing unit. In this case the person 2 or another person like a nurse needs to position the person 2 such that the posture is in correspondence with the predefined stored posture. Moreover, in another embodiment the posture providing unit itself can be adapted to allow a user to input the current posture of the person 2.

In this embodiment the posture providing unit 5 is adapted to provide the posture of the person 2 by providing an angle between the person's back and a horizontal plane, while the person lies on the person's back. This angle could also be regarded as being a reclination angle of a supine posture.

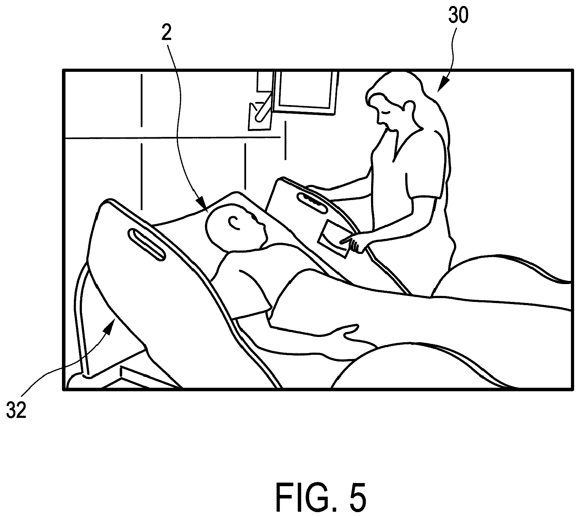

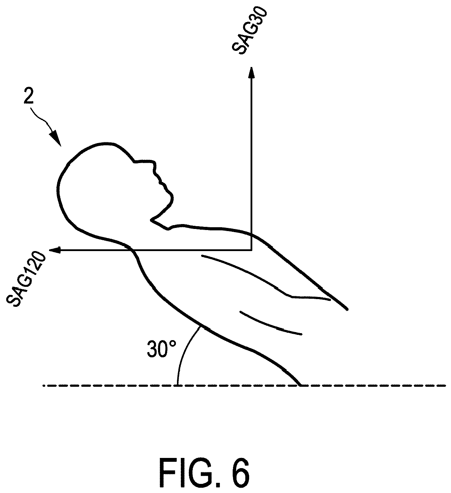

Fig. 5 schematically and exemplarily illustrates that a nurse 30 might modify the inclination of an upper part of a bed 32 on which the person 2 might be lying in the supine posture, wherein the system 1 is horizontally attached to the left side of the chest of the person 2 as schematically illustrated in Fig. 1. In this embodiment the bed angle or reclination angle is 30 degrees, i.e. the upper part of the bed 32 encloses an angle of 30 degrees with the horizontal base of the bed. The angle of 30 degrees is also illustrated in Fig. 6 which shows the upper inclined part of the person 2 in the sagittal plane.

The device 4 further comprises a transformation determination unit 6 adapted to determine a transformation between the coordinate system 8 of the accelerometer 3 and the coordinate system 9 of the subject 2, wherein this determination of the transformation includes a determination of the direction of the acceleration in the coordinate system 8 of the accelerometer 3, if the person 2 does not move. Since the acceleration is measured with respect to the earth's gravitational field, this direction is vertical and points upward. The determination of the transformation further includes determining a vertical and upward pointing anatomical direction SAG30 in the sagittal plane in the coordinate system 9 of the person 2, which is illustrated in Fig. 6, based on the provided reclination angle of the supine posture and defining that the vertical and upward pointing anatomical direction SAG30 in the sagittal plane in the coordinate system 9 of the person 2 is equal to the direction of the acceleration in the coordinate system 8 of the accelerometer 3, if the subject 2 does not move, in order to define a first direction in the coordinate system 8 of the accelerometer 3. Since in this embodiment the bed angle of 30 degrees is known, the vertical, upward pointing anatomical direction in the sagittal plane SAG30 in the coordinate system 9 of the person 2 can be determined based on the provided reclination angle of the supine posture, i.e. the provided bed angle of 30 degrees in the sagittal plane. The determination of the transformation further includes providing a second direction in the coordinate system 8 of the accelerometer 3 being equal to a direction in the coordinate system 9 of the person 2 having a known spatial relation to the vertical and upward pointing anatomical direction SAG30 in the sagittal plane in the coordinate system 9 of the person 2. In this embodiment the transformation determination unit 6 is adapted to provide the second direction such that it is equal to the medial-lateral direction ML in the coordinate system 9 of the person 2. The known spatial relation is correspondingly an orthogonal relation. The determination of the transformation further includes modifying the first and second directions such that they correspond to the known spatial relation, i.e. in this embodiment to the orthogonality, and determining the transformation based on the modified first and second directions and the known spatial relation. The transformation determination unit 6 can be further adapted to determine, before modifying the first and second directions such that they correspond to the known spatial relation, whether a deviation from the known spatial relation is smaller than a predefined threshold and to stop the determination of the transformation, if the deviation is not smaller than the predefined threshold.

The device 4 further comprises an electrocardiography signal providing unit 11 adapted to generate an electrocardiography signal of the person 2 having a polarity sign, wherein in this embodiment the electrocardiography signal providing unit 11 is adapted to actually generate the electrocardiography signal. In another embodiment the electrocardiography signal providing unit can be adapted to receive the electrocardiography signal from a separate electrocardiography signal generating unit. The transformation determination unit 6 is adapted to provide the sign of the second direction based on the provided polarity sign. In another embodiment the transformation determination unit 6 can also be adapted to determine the sign of the second direction based on an acceleration value received from the accelerometer 3.

Since the first and second directions are known in the coordinate system 9 of the person 2 and since they have been defined with respect to the coordinate system 8 of the accelerometer 3, these directions already represent a transformation between these two coordinate systems 8, 9. A third direction can be defined by a cross product of the first and second directions.

The system 1 further comprises a condition determination unit 14 for determining a condition of the person 2 based on an acceleration measured by the accelerometer 3, after the transformation has been determined, i.e. after the transformation determination procedure, which might also be regarded as being a calibration procedure, has been completed, and based on the determined transformation. In particular, the condition determination unit 14 is adapted to transform the measured acceleration from the coordinate system 8 of the accelerometer 3 to the coordinate system 9 of the person 2, wherein the condition determination unit 14 determines the condition of the person 2 based on the transformed acceleration. The condition determination unit 14 can be adapted to use known algorithms which are adapted to determine a condition of a subject based on a measured acceleration. For instance, the condition determination unit 14 can be adapted to determine the posture of the person 2 based on the transformed acceleration. However, the condition determination unit 14 can also be adapted to determine another condition of the person 2 like the activity of the person 2.

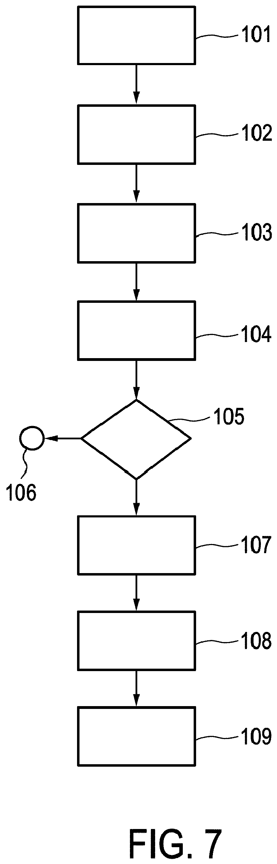

In the following an embodiment of a method for determining a spatial relation between a coordinate system of an accelerometer attached to a person and a coordinate system of the person will exemplarily be described with reference to a flowchart shown in Fig. 7.

In step 101 the posture of the person is provided by the posture providing unit 5. For instance, an angle of 30 degrees in a sagittal plane between the posterior-anterior direction $ANT$ and a vertical line is provided as the posture of the person 2. In step 102 the direction $GCA$ of the acceleration in the coordinate system 8 of the accelerometer 3 is determined, while the person 2 does not move. Moreover, in step 102 a vertical and upward pointing direction $SAG30$ is determined in the coordinate system of the subject based on the posture provided in step 102. In step 103 it is defined that the vertical and upward pointing direction $SAG30$ in the coordinate system of the subject is equal to the direction $GCA$ of the acceleration in the coordinate system 8 of the accelerometer 3, if the person 2 does not move, in order to define the first direction in the coordinate system 8 of the accelerometer 3.

In step 104 a second direction is provided in the coordinate system 8 of the accelerometer 3 being equal to a direction in the coordinate system 9 of the person 2 having a known spatial relation to the vertical and upward pointing direction in the coordinate system 9 of the person 2. In particular, the second direction in the coordinate system 8 of the accelerometer 3 is provided such that it is equal to the medial-lateral direction $ML$ in the coordinate system 9 of the person 2, wherein in this case the known spatial relation is an orthogonal spatial relation. In step 105 it is determined whether a deviation from the known spatial relation is smaller than a predefined threshold, wherein, if this is not the case, the method stops in step 106. Moreover, in step 105 or 106, it may be output to a user that the determination of the transformation, i.e. the calibration of the system 1, cannot be carried out. For instance, in step 105 it can be checked whether the angle between the first and second directions is within a range of 60 to 120 degrees, if the known spatial relation between these two directions should be an orthogonal spatial relation. If the deviation from the known spatial relation is smaller than a predefined threshold, the method continues with step 107. In step 107 the first and second directions are modified such that they correspond to the known spatial relation, i.e., for instance, such that they enclose an angle of 90 degrees if the known spatial relation is an orthogonal spatial relation. In step 108 a third direction is determined as a cross product of the first and second directions, and in step 109 the transformation is determined based on the first, second and third directions.

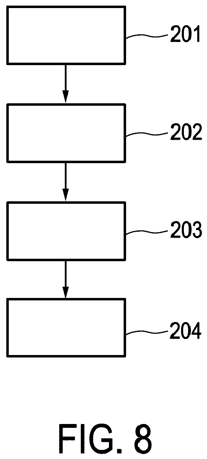

In the following an embodiment of a method for determining a condition of a subject will exemplarily be described with reference to a flowchart shown in Fig. 8.

In step 201 a first acceleration is measured by the accelerometer 3 attached to the person 2, while the person 2 does not move. In step 202 a transformation between the coordinate system 8 of the accelerometer 3 and the coordinate system 9 of the person 2 is determined based on the measured first acceleration as described above with reference to Fig. 7. In step 203, i.e. after the determination of the transformation and hence the calibration has been completed, a second acceleration is measured by the accelerometer 3, wherein in step 204 a condition of the person 2 like the posture of the person is determined based on the measured second acceleration and the determined transformation by the condition determination unit 11. In particular, the measured second acceleration is transformed from the coordinate system 8 of the accelerometer 3 to the coordinate system 9 of the person 2 and the condition like the posture or the activity is determined based on the transformed second acceleration.

Monitoring body posture can be important, for instance, in order to prevent decubitus. Body postures that can be monitored are, for example, lying reclined, supine, prone, on the left side, on the right side, being upright, i.e. sitting or standing, et cetera.

The posterior-anterior direction, i.e. the corresponding posterior-anterior axis ANT, is preferentially defined such that it is exactly horizontally from back to front when the subject is standing upright and exactly vertical if the person is lying on his back.

The determined transformation is a determination of how the body axes of the subject, i.e. the coordinate system of the subject, are oriented with respect to the accelerometer, i.e. the coordinate system of the accelerometer, and vice versa. This determination of the transformation or relative orientation is regarded as being the calibration of the system. The determined transformation can be regarded as being a rotation matrix which converts the sensing axes of the tri-axial accelerometer 3 into the anatomical directions of the person. The calibration is not only useful for determining the posture, i.e. for determining the body posture, but also for determining other conditions of the subject like the activity such as step detection and fall detection. Thus, the calibrated system can also be used for activity monitoring. Due to the calibration the condition like the posture or the activity can be determined with a higher sensitivity and specificity. The above described calibration requires a minimal time and effort from, for instance, the nurse. Moreover, the calibration can be carried out very quickly such that the sensor can be used for the actual condition monitoring, for instance, the actual posture monitoring or activity monitoring very quickly after the system has been attached to the subject. Furthermore, the calibration does not require any effort from the person, which might be a patient, such that the person is not stressed.

The accelerometer is preferentially a three-axes accelerometer that is to be attached on a subject, preferably a living being, i.e. a human being or an animal, and preferably on the thorax of a human being, especially on the left side of the chest of the human being. However, the subject can also be a non-living subject like a robot, i.e. also to a non-living subject like a robot the accelerometer can be attached for determining, for instance, the posture and/or the activity of the non-living subject. The determination of the activity can include, for instance, whether the subject has fallen, has exited the bed, walks freely, walks with a support like a walking stick, a crutch or a rollator, whether the subject sleeps, whether the subject cycles, et cetera.

The device 4 is adapted to determine whether the system with the accelerometer has been placed upside down and, if this is the case, the system does not need to be reapplied, but the system itself, i.e. the above described algorithm, can cope with this situation by detecting whether the system has been placed upside down and by considering this knowledge when determining the sign of the second direction. In particular, as will be explained further below, the $y$ axis of the coordinate system of the accelerometer, which preferentially corresponds to the length axis of the system 1, can be multiplied with -1, in order to consider an upside down placement of the system 1 and hence of the accelerometer. For determining whether the system 1 and hence the accelerometer has been placed upside down on the subject a measurement of the acceleration in the coordinate system of the accelerometer or an electrocardiography signal can be used, especially the polarity of the electrocardiography signal. If the polarity of the electrocardiography signal is opposite to what is expected, if the system and hence the accelerometer were placed correctly on the subject, i.e. not upside down, the system and hence the accelerometer have been placed upside down. However, as mentioned above, the system with the accelerometer does not need to be reapplied, because the algorithm can cope with this by, for instance, multiplying the length axis of the system 1 and hence the $y$ axis of the coordinate system of the accelerometer with -1. Exploiting the polarity of the electrocardiography signal is preferred, because, if the outward pointing axis of the coordinate system of the accelerometer, i.e. the z axis, approaches alignment or is aligned with the gravity vector during the calibration, the acceleration, i.e. the gravity vector, cannot be used for determining whether the system 1 and hence the accelerometer has been placed upside down. For more details regarding the use of the electrocardiography signal for determining how the system and hence the accelerometer have been attached to the subject reference is made to WO 2017/191036 A1 which is herewith incorporated by reference.

In the embodiment described above with reference to Figs. 1 to 8 it is assumed that the person is lying on his/her back in a bed, i.e. in a supine posture, wherein the bed angle and hence the angle that the person's back makes with the horizontal is provided by the posture providing unit. In a practical situation this can mean that the posture providing unit always provides the same predefined stored bed angle of, for instance, 30°, wherein, for example, a nurse is given an instruction to apply the system with the accelerometer to the person while the person is lying on his back in the bed under the given bed angle. In the following it is assumed that this bed angle, i.e. the angle that the person's back makes with the horizontal, is 30°. However, in general it could be any angle $\theta$. For this more general case in the following a reference to 30° can be replaced by $\theta$, 120° can be replaced by 90°+$\theta$, sagittal-30 can be replaced by sagittal-$\theta$ and sagittal-120 can be replaced by sagittal-(90°+$\theta$). If in an embodiment the bed angle is not 30° and settable by, for instance, a nurse, an app on the above described input device 22 could be adapted to allow the nurse to input the current bed angle. The bed itself can also comprise an inclination sensor that communicates its angle to the posture providing unit which receives the sensed bed angle and which provides the sensed bed angle for the further processing. It is also possible to use a two-step approach, wherein in a first step the system with the accelerometer is placed on the bed, wherein in this case the transformation determination unit can be adapted to, in a first step, determine the bed angle and wherein in a second step this sensed bed angle can be used for the calibration procedure. For determining the bed angle in the first step the nurse can be instructed to place the system on the upper bed plane such that the z axis of the coordinate system of the accelerometer is vertically oriented, if the bed angle is zero, wherein then, if the bed angle is modified, the orientation of the gravitational vector within the coordinate system of the accelerometer can be used for determining the bed angle. Moreover, the nurse or another person placing the system with the accelerometer on the subject is preferably instructed to apply the system with its length axis, i.e. with its y axis, in the medial-lateral direction ML as schematically and exemplarily illustrated in Fig. 1. However, the system itself, i.e. the algorithm applied by the system, can compensate for slight variations on this system direction, as will be explained further below.

The direction SAG30 in the coordinate system of the person 2 can be regarded as being a combined anatomical direction which is in the sagittal plane between the caudal-cranial direction CR and the posterior-anterior direction ANT, wherein this combined anatomical direction makes an angle of 30° with the posterior-anterior direction ANT and an angle of 60° with the caudal-cranial direction CR. This combined anatomical direction can also be regarded as being a sagittal-30 direction or short SAG30 direction. Moreover, a sagittal-120 or short SAG120 direction is defined, which has an angle of 120° with the posterior-anterior direction ANT. The SAG30 direction and the SAG120 direction are both illustrated in Fig. 6. The implication of the current requirement of applying the sensor, i.e. the system with the accelerometer, with a bed angle of 30° is that the acceleration signal measured directly after applying the system is approximately aligned with the SAG30 direction.

It is assumed that, as illustrated in Fig. 2, the accelerometer 3 is placed with its z axis pointing "forward" in the height direction out of the system 1, the y axis along the longest direction of the system 1 and the x axis perpendicular to both, the y axis and the z axis. In case the accelerometer 3 is placed in a different way within the system 1, this can be easily compensated by using a rotation matrix from the coordinate system of the accelerometer to a reference frame or coordinate system of the system 1, especially of the housing 20 of the system 1. It is just required that the spatial relation between the accelerometer and a housing of the system, i.e. the placement of the accelerometer within the system, is known.

The calibration carried out by the transformation determination unit 6 preferentially leads to a rotation matrix from the accelerometer x, y and z axes to the caudal-cranial axis CR, the posterior-anterior axis ANT and the medial-lateral direction ML.

These anatomical directions CR, ANT, ML are preferentially determined in the coordinate system of the accelerometer, which results in following rotation matrix $R_{cal}$:

$$R_{\mathrm{cal}} = \begin{bmatrix} ML_x & CR_x & ANT_x \\ ML_y & CR_y & ANT_y \\ ML_z & CR_z & ANT_z \end{bmatrix}. \qquad (1)$$

The system can be adapted to start the calibration automatically as soon as the system has been applied to the skin of the person as from then on an electrocardiography signal is available, which can be used as "on-skin" detection. It is also possible that the system is adapted to provide another form of on-skin detection based on, for instance, a measurement of a heart rate in a predefined range like in between 30 and 120 bpm. For this heart rate measurement the accelerometer signal can be used. The system can also comprise a further sensor for measuring the heart rate like a PPG sensor. The system can also be adapted to start the calibration on demand. For instance, a start signal can be provided via the input unit 22 or the system might comprise a button or the like which might be pressed by, for instance, a nurse for starting the calibration procedure.

The system is preferentially further adapted to check whether the person does not move, for instance, is lying sufficiently still such that the calibration procedure can start. For instance, the transformation determination unit can be adapted to monitor the variation in the acceleration signal measured by the accelerometer, wherein, if this variation is sufficiently low, i.e., for instance, smaller than a predefined threshold, the measured acceleration is dominated by an acceleration with the magnitude of 1 g being the acceleration with respect to gravity of a device that is not moving. When indeed the person is lying still, the measured acceleration, which might be averaged over a certain time like a couple of seconds, within the coordinate system of the accelerometer will be a vertical and upwards pointing direction which could be named gravity canceling acceleration (GCA). This gravity canceling acceleration, i.e. the direction GCA, is used during the calibration, i.e. during the determination of the transformation between the coordinate system of the accelerometer and the coordinate system of the person. If the person turns out to be moving, the previous steps can be repeated until stable acceleration is measured.

In the preferred embodiment the length axis of the system 1 is aligned with the accelerometer $y$ axis. The length axis $SL$ of the system 1 is thus preferentially defined as a vector $SL = [0\ 1\ 0]$ in the coordinate system 8 of the accelerometer 3. If the accelerometer 3 is mounted differently in the system 1, this vector is preferentially adjusted.

In an embodiment in a first step the electrocardiography polarity sign is determined, in order to deal with the possibility that the system 1 and hence the accelerometer 3 is applied reversed. This can give a sign $P = 1$ if applied correctly, and $P = -1$ if applied reversed. In a next, second step, as a first estimate of the anatomical-left direction, i.e. of the medial-lateral direction $ML$, in the coordinate system 8 of the accelerometer 3 the system's length axis $SL$ times the polarity sign is determined. This approximation of the anatomical-left direction $ML$ in the coordinate system 8 of the accelerometer 3, i.e. of the second direction, is based on the assumption that the system 1 is applied always in the same location and orientation. This first estimate $ML_1$ of the second direction $ML$ might thus be described by $ML_1 = P \cdot DL$.

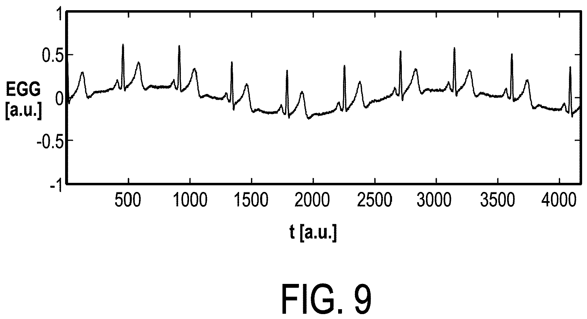

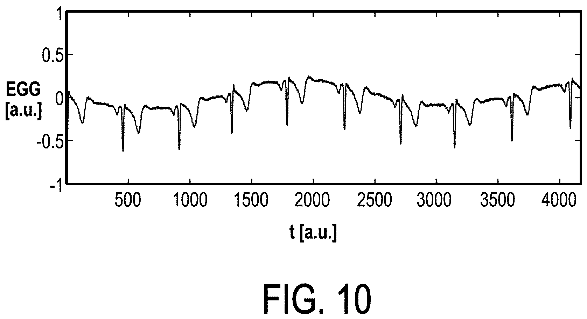

The first and second steps take care of a system and hence accelerometer that is applied upside down, i.e. - in case the system 1 looks like that of Fig. 2 - with the letters of the logo upside down from the view of, for instance, the nurse. The change of the polarity of the electrocardiography signal when the system 1 is place upside down is illustrated in Figs. 9 und 10 showing electrocardiography signals EEG versus time, both in arbitrary units. In Fig. 9 the system 1 is placed "normally", i.e. as it should be placed in accordance with given instructions like that the logo should be visible, and in Fig. 10 the system 1 is placed upside down. Without taking this electrocardiography polarity into account, lying on the left side would be seen as lying on the right side and vice versa and standing/sitting upright would be seen as being upside down, i.e. head down, if the system 1 were placed upside down.

Instead of using the electrocardiography polarity for the upside down detection, the accelerometer 3 could be used. However, the accelerometer solution will not work for all bed angles $\theta$. Especially for $\theta$ being close to zero, the accelerometer-based solution might not work. For angles sufficiently far from zero, the acceleration measured in the system's height axis, which is the accelerometer's $x$ axis if the accelerometer 3 is placed in the system 1 as shown in Fig. 2, could be used to determine whether the device has been placed upside down, and if so, like for the electrocardiography-polarity-check solution, $P$ should be set to -1.

In a third step a first estimate $SAG30_1$ of the sagittal-30 direction, i.e. the first direction, in the coordinate system 8 of the accelerometer 3 is determined as the normalized direction of the gravity-cancelling acceleration, i.e. $SAG30_1 = GCA / \mathrm{norm}(GCA)$, wherein in a fourth step it is checked whether the first estimates of $SAG30$ and $ML$ directions, i.e. the first and second directions, are approximately orthogonal. That means it is checked, for instance, whether the angle between $ML_1$ and $SAG30_1$ is between 60 and 120 degrees or whether the absolute value of their dot product is smaller than 0.5. For example, if this dot product $x = \mathrm{dot}(ML_1, SAG30_1)$ is smaller than 0.5, the calibration procedure proceeds, wherein, if this dot product is equal to or larger than 0.5, the calibration is preferentially stopped, because the calibration would be invalid. The fourth step thus checks whether indeed the length system direction, which in this embodiment is equal to the direction of the $y$ axis of the coordinate system 8 of the accelerometer 3 because the accelerometer 3 and hence the coordinate system 8 is placed in the system as indicated in Fig. 2, is approximately orthogonal to the sagittal plane like it should be for a system being placed in the way of placement as shown in Fig. 1. When they are far from orthogonal, the system 1 is preferentially adapted to give as output that the calibration failed and the calibration process is stopped. If not, the calibration continues with the next step being the fifth step in this example.

In the fifth step the first estimates are adjusted, i.e. the first and second directions are modified, to enforce orthogonality. This modification of the first and second directions preferentially uses a correction factor $\varepsilon$ that depends on the dot product. In particular, the correction factor is defined by $\varepsilon = (-1 + \mathrm{sqrt}(1 - x^2))/x$. The modification can be defined by:

$$ML = (ML_1 + \varepsilon \cdot SAG30_1)/\mathrm{sqrt}(1 + \varepsilon^2 + 2 \cdot \varepsilon \cdot x) \qquad (2)$$

and by

$$SAG30 = (SAG30_1 + \varepsilon \cdot ML_1)/\mathrm{sqrt}(1 + \varepsilon^2 + 2 \cdot \varepsilon \cdot x). \qquad (3)$$

In this fifth step a correction to accurately determine the medial-lateral direction $ML$ takes place when the two directions are not exactly orthogonal. This correction assigns the orthogonality error evenly over the $ML$ and $SAG30$ directions. This might be generalized by a weighting factor w between 0 and 2, indicating the weight of the assigned correction of the $ML$ direction. The weighting factor may be a fixed value, or depend on features of the detected electrocardiography or acceleration signals. Introducing the weighting factor would change the fifth step to:

$$\varepsilon = (-1 + \mathrm{sqrt}(1 - 1 \cdot w \cdot (2 - w) \cdot x^2)) / (w \cdot (2 - w) \cdot x), \qquad (4)$$

$$ML = (ML_1 + w \cdot \varepsilon \cdot SAG30_1) / \mathrm{sqrt}(1 + (w \cdot \varepsilon)^2 + 2 \cdot w \cdot \varepsilon \cdot x)$$

$$(5)$$

and

$$SAG30 = SAG30_1 + (2 - w) \cdot \varepsilon \cdot ML_1) / \mathrm{sqrt}(1 + ((2 - w) \cdot \varepsilon)^2 + 2 \cdot (2 - w) \cdot \varepsilon \cdot x).$$

$$(6)$$

To derive these formulas, it is assumed that the best estimate of the correct *ML* and *SAG*30 directions lies in the same plane as the $ML_1$ and $SAG30_1$ directions, wherein the suffix '1' stands for the originally measured directions. This implies that *ML* and *SAG*30 can be written as a linear combination of the $ML_1$ and $SAG30_1$ directions, i.e.

$$ML = (ML_1 + w \cdot \varepsilon \cdot SAG30_1) / l1 \qquad \text{and} \qquad (7)$$

$$SAG30 = (SAG30_1 + (2 - w) \cdot \varepsilon \cdot ML_1) / l2 , \qquad (8)$$

where *l*1 and *l*2 are the lengths of the vectors and are used to normalize the vectors to unit length. The value of $\varepsilon$ can be determined by setting the dot product of the resulting directions to 0, i.e.

$$\mathrm{dot}(ML, SAG30) = 0, \qquad (9)$$

$$\mathrm{dot}(ML_1 + w \cdot \varepsilon \cdot SAG30_1, SAG30_1 + (2 - w) \cdot \varepsilon \cdot ML_1) = 0$$

$$(10)$$

and

$$\mathrm{dot}(ML_1, SAG30_1) + w \cdot \varepsilon \cdot \mathrm{dot}(SAG30_1, SAG30_1) +$$

$$(2 - w) \cdot \varepsilon \cdot \mathrm{dot}(ML_1, ML_1) + w \cdot \varepsilon \cdot (2 - w) \cdot \varepsilon \cdot \mathrm{dot}(SAG30_1, ML_1) = 0 ,$$

$$(11)$$

after filling in the values for the dot products:

$$x + w \cdot \varepsilon \cdot 1 + (2 - w) \cdot \varepsilon \cdot 1 + w \cdot \varepsilon \cdot (2 - w) \cdot \varepsilon \cdot x = 0 ,$$

$$(12)$$

$$w \cdot (2 - w) \cdot x \cdot \varepsilon^2 + 2 \cdot \varepsilon + x = 0 ,$$

$$(13)$$

$$\varepsilon = (-2 \pm \text{sqrt}(4 - 4 \cdot w \cdot (2 - w) \cdot x \cdot x)) / (2 \cdot w \cdot (2 - w) \cdot x)$$

$$\varepsilon = (-1 \pm \text{sqrt}(1 - 1 \cdot w \cdot (2 - w) \cdot x \cdot x)) / (w \cdot (2 - w) \cdot x) \qquad .$$

$$(14)$$

and

The "+" option is preferentially selected, in order to keep the correction as small as possible, i.e.

$$\varepsilon = (-1 + \text{sqrt}(1 - 1 \cdot w \cdot (2 - w) \cdot x^2)) / (w \cdot (2 - w) \cdot x)$$

$$(15)$$

By setting $l1$ and $l2$ equal to the lengths of the above two linear combinations, the lengths of the direction vectors is set to 1, i.e.

$$l1 = \text{sqrt}(\text{dot}(ML_1 + w \cdot \varepsilon \cdot SAG30_1, ML_1 + w \cdot \varepsilon \cdot SAG30_1)) ,$$

$$(16)$$

$$l1 = \text{sqrt}(dot(ML_1, ML_1) + (w \cdot \varepsilon)^2 \cdot \text{dot}(SAG30_1, SAG30_1) +$$

$$2 \cdot w \cdot \varepsilon \cdot \text{dot}(ML_1, SAG30_1)) \quad \text{and}$$

$$(17)$$

$$l1 = \text{sqrt}(1 + (w \cdot \varepsilon)^2 + 2 \cdot w \cdot \varepsilon \cdot x)$$

$$(18)$$

In the same way

$$l2 = \text{sqrt}(1 + ((2 - w) \cdot \varepsilon)^2 + 2 \cdot (2 - w) \cdot \varepsilon \cdot x)$$

$$(19)$$

is obtained. By this, the calibration is fine-tuned, i.e. by including this step in the calibration, the exact orientation of the subject's body will be more accurately measured after the calibration has finished.

In a sixth step the sagittal-120 direction is determined as $SAG120 = \text{cross}(SAG30, ML)$. In a seventh step the cosine and sine of the inclination angle of 30 degrees are used, i.e. $\cos(30) = 0.5 \cdot \text{sqrt}(3)$ and $\sin(30) = 0.5$, and in a eighth step the caudal-cranial and posterior-anterior directions $CR$ and $ANT$ in the coordinate system 8 of the accelerometer 3 are determined as combinations of $SAG30$ and $SAG120$ in accordance with

$$CR = \cos(30) \cdot SAG120 + \sin(30) \cdot SAG30 \qquad (20) \quad \text{and}$$

$$ANT = \cos(30) \cdot SAG30 - \sin(30) \cdot SAG120 \quad . \qquad (21)$$

In these steps the found directions are used to determine the caudal-cranial direction *CR* and the posterior-anterior direction *ANT.* These directions together with the determined medial-lateral direction *ML* in the coordinate system 8 of the accelerometer 3 define the transformation, i.e., for instance, as described by equation (1).

The eighth step including the determination of the rotation matrix in accordance with equation (1) ends the calibration process, and from then on, the system can output, for example, the postures of the person in the bed or another condition of the person.

The system 1 can be regarded as being a wearable for posture and/or activity tracking. The system 1 is adapted for being chest-worn. However, in another embodiment the system can also be adapted to be, for instance, belly-worn. Especially the monitoring of body posture can be valuable for the prevention of pressure ulcer.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the provision of the posture of the subject, the determination of the transformation, the determination of the condition, et cetera performed by one or several units or devices can be performed by any other number of units or devices. These procedures and/or the control of the device for determining the transformation between the coordinate system of the accelerometer attached to a subject and the coordinate system of the subject in accordance with the method for determining the transformation between the coordinate system of the accelerometer attached to the subject and the coordinate system of the subject and/or the control of the system for determining a condition of the subject like its posture and/or its activity in accordance with the method for determining the condition of the subject can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a device for determining a transformation between an accelerometer coordinate system of an accelerometer attached to a subject and a subject coordinate system. Defining that a vertical and upward pointing direction in the subject coordinate system is equal to an acceleration-measurement-based vertical and upward pointing direction in the accelerometer coordinate system leads to a first direction in the accelerometer coordinate system. A second direction in the accelerometer coordinate system is provided, which is equal to a direction in the subject coordinate system of the subject having a known spatial relation to the vertical and upward pointing direction in the subject coordinate system, wherein the transformation is determined based on the first and second directions and the known spatial relation. This allows for an improved determination of the transformation, which can be used for, for instance, improved activity or posture detection.

## Claims

1. A device for determining a transformation between a coordinate system of an accelerometer (3) attached to a subject (2) and a coordinate system (9) of the subject (2), wherein the device (4) comprises:

   a transformation determination unit (6) adapted to determine a transformation between the coordinate system (8) of the accelerometer (3) and the coordinate system (9) of the subject (2), wherein the determination of the transformation includes:

   a) determining a vertical and upward pointing direction (*GCA*) in the coordinate system (8) of the accelerometer (3) based on an acceleration measured by the accelerometer, providing a vertical and upward pointing direction (*SAG*30) in the coordinate system of the subject and defining that the vertical and upward pointing direction (*SAG*30) in the coordinate system of the subject is equal to the vertical and upward pointing direction in the coordinate system (8) of the accelerometer (3), in order to define a first direction in the coordinate system of the accelerometer,
   b) providing a second direction in the coordinate system of the accelerometer being equal to a direction in the coordinate system of the subject (2) having a known spatial relation to the vertical and upward pointing direction in the coordinate system of the subject,
   c) determining the transformation based on the first and second directions and the known spatial relation.

2.  The device as defined in claim 1, wherein the transformation determination unit (6) is adapted to provide the second direction such that it is equal to the medial-lateral direction in the coordinate system of the subject (2).

3.  The device as defined in claim 1, wherein the transformation determination unit (6) is adapted to determine the transformation based on the first and second directions such that it includes modifying the first and second directions such that they correspond to the known spatial relation and determining the transformation based on the modified first and second directions and the known spatial relation.

4.  The device as defined in claim 3, wherein the known spatial relation is an orthogonal spatial relation, wherein the transformation determination unit (6) is adapted to modify the first and second directions such that they are orthogonal to each other.

5.  The device as defined in claim 3, wherein transformation determination unit (6) is further adapted to determine, before modifying the first and second directions such that they correspond to the known spatial relation, whether a deviation from the known spatial relation is smaller than a predefined threshold and to stop the determination of the transformation, if the deviation is not smaller than the predefined threshold.

6.  The device as defined in claim 1, wherein the device (4) further comprises an electrocardiography signal providing unit (11) adapted to generate an electrocardiography signal of the subject (2) having a polarity sign, wherein the electrocardiography signal is generated by an electrocardiography signal generating unit having a fixed spatial relation to the accelerometer, wherein the transformation determination unit (6) is adapted to provide the sign of the second direction based on the provided polarity sign.

7.  The device as defined in claim 1, wherein the transformation determination unit (6) is adapted to determine the sign of the second direction based on an acceleration value received from the accelerometer (3).

8.  The device as defined in claim 1, wherein the device further comprises a posture providing unit (5) adapted to provide the posture of the subject (2), wherein the transformation determination unit is adapted to provide the vertical and upward pointing direction in the coordinate system of the subject (2) based on the provided posture.

9.  The device as defined in claim 8, wherein the posture providing unit (5) is adapted to provide a predefined stored posture as the posture of the subject (2) and/or to provide a user interface for allowing a user to input the posture of the subject (2) into the device and/or to receive a user input indicating the posture of the subject (2) from an input device (22) at which the user has input the posture, wherein the posture providing unit (5) is adapted to provide the input posture.

10. The device as defined in claim 8, wherein the accelerometer is adapted to be attached to a chest of the subject (2), wherein the posture providing unit (5) is adapted to provide the posture of the subject (2) by providing an angle between the subject's back and a horizontal plane, while the subject lies on the subject's back, wherein the transformation determination unit (6) is adapted to determine the vertical and upward pointing direction in the coordinate system of the subject (2) in the sagittal plane based on the provided angle between the subject's back and the horizontal plane.

11. A system for determining a condition of a subject, wherein the system (1) comprises:

    an accelerometer (3) adapted to be attached to the subject (2) and to measure a first acceleration, while the subject (2) does not move, and a second acceleration,
    a device (4) as defined in claim 1 for determining a transformation between a coordinate system (8) of the accelerometer (3) and a coordinate system (9) of the subject (2), when the accelerometer (3) is attached to the subject (2), wherein the first acceleration is used for determining the vertical and upward pointing direction in the coordinate system of the accelerometer, and
    a condition determination unit (14) for determining a condition of the subject (2) based on the measured second acceleration and the determined transformation.

12. A method for determining a transformation between a coordinate system of an accelerometer (3) attached to a subject (2) and a coordinate system (9) of the subject (2), wherein the method comprises:

    determining a transformation between the coordinate system (8) of the accelerometer (3) and the coordinate system (9) of the subject (2) by a transformation determination unit (6), wherein the determination of the transformation includes:

a) determining a vertical and upward pointing direction (*GCA*) in the coordinate system (8) of the accelerometer (3) based on an acceleration measured by the accelerometer, providing a vertical and upward pointing direction (*SAG*30) in the coordinate system of the subject and defining that the vertical and upward pointing direction (*SAG*30) in the coordinate system of the subject is equal to the vertical and upward pointing direction in the coordinate system (8) of the accelerometer (3), in order to define a first direction in the coordinate system of the accelerometer,

b) providing a second direction in the coordinate system of the accelerometer being equal to a direction in the coordinate system of the subject (2) having a known spatial relation to the vertical and upward pointing direction in the coordinate system of the subject,

c) determining the transformation based on the first and second directions and the known spatial relation.

13. A method for determining a condition of a subject, wherein the method comprises:

measuring a first acceleration by an accelerometer (3) attached to the subject (2), while the subject (2) does not move,

determining a transformation between a coordinate system (8) of the accelerometer (3) and a coordinate system (9) of the subject (2) based on the measured first acceleration as defined in claim 12, wherein the measured first acceleration is used for determining the vertical and upward pointing direction in the coordinate system of the accelerometer,

measuring a second acceleration by the accelerometer (3) attached to the subject (2),

determining a condition of the subject (2) based on the measured second acceleration and the determined transformation by a condition determination unit (11).

14. A computer program for determining a transformation between a coordinate system of an accelerometer attached to a subject and a coordinate system of the subject, the computer program comprising program code means for causing a device as defined in claim 1 to carry out the steps of the method as defined in claim 12, when the computer program is run on a computer controlling the device.

15. A computer program for determining a condition of a subject, the computer program comprising program code means for causing a system as defined in claim 11 to carry out the steps of the method as defined in claim 13, when the computer program is run on a computer controlling the system.

FIG. 1

FIG. 2

14 □          □ 3

5 □    □    □ 6

22 □    ┄┄→    □ 11    4

# FIG. 3

FIG. 4

FIG. 5

FIG. 6

FIG. 7

201

202

203

204

FIG. 8

FIG. 9

**FIG. 10**

Europäisches
Patentamt

European
Patent Office

Office européen
des brevets

**EUROPEAN SEARCH REPORT**

Application Number

EP 18 15 0136

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/115277 A1 (WANG HUA [US] ET AL) 24 May 2007 (2007-05-24) * paragraphs [0028] - [0038], [0043], [0050] - [0056], [0070] - [0072], [0096], [0097], [0100] * | 1-15 | INV. A61B5/11 ADD. A61B5/0402 A61B5/00 G01C25/00 |
| X | US 2014/296660 A1 (VAN DE LAAR JAKOB [NL] ET AL) 2 October 2014 (2014-10-02) * paragraphs [0030], [0031], [0073] - [0095] * | 1,12 | |
| A | US 2013/116602 A1 (VAN DEN HEUVEL TEUN [NL] ET AL) 9 May 2013 (2013-05-09) * the whole document * | 1-15 | |

TECHNICAL FIELDS
SEARCHED      (IPC)

A61B
G01C

The present search report has been drawn up for all claims

| Place of search | Date of completion of the search | Examiner |
|---|---|---|
| The Hague | 6 July 2018 | Kowalczyk, Szczepan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.                    EP 18 15 0136

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2018

| Patent document cited in search report | Publication date | Patent family member(s) | Publication date |
|---|---|---|---|
| US 2007115277 A1 | 24-05-2007 | NONE | |
| US 2014296660 A1 | 02-10-2014 | BR 112014008950 A2 | 02-05-2017 |
| | | CN 103889325 A | 25-06-2014 |
| | | EP 2747648 A1 | 02-07-2014 |
| | | JP 6014153 B2 | 25-10-2016 |
| | | JP 2014533140 A | 11-12-2014 |
| | | MX 344427 B | 15-12-2016 |
| | | RU 2014119854 A | 27-11-2015 |
| | | US 2014296660 A1 | 02-10-2014 |
| | | WO 2013057622 A1 | 25-04-2013 |
| US 2013116602 A1 | 09-05-2013 | CN 103025240 A | 03-04-2013 |
| | | EP 2598028 A2 | 05-06-2013 |
| | | JP 5847178 B2 | 20-01-2016 |
| | | JP 2013532539 A | 19-08-2013 |
| | | US 2013116602 A1 | 09-05-2013 |
| | | WO 2012014110 A2 | 02-02-2012 |

EPO FORM P0459

For more details about this annex : see Official Journal of the European Patent Office, No. 12/82

**EP 3 508 121 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9035794 B2

- WO 2017191036 A1